# EUROPEAN PATENT SPECIFICATION

(11) **EP 4 154 932 B1**
(45) Date of publication and mention of the grant of the patent: **24.04.2024**
(21) Application number: 20736389.6
(22) Date of filing: 18.05.2020
(51) Int. Cl.: A61M 16/04

(54) **ENDOTRACHEAL DEVICE FOR MECHANICAL VENTILATION OF A PATIENT**
ENDOTRACHEALVORRICHTUNG ZUR MECHANISCHEN BEATMUNG EINES PATIENTEN
DISPOSITIF ENDOTRACHÉAL POUR VENTILATION MÉCANIQUE D'UN PATIENT

(43) Date of publication of application: 29.03.2023
(73) Proprietor: BRAVO GARCÍA, Pedro Luis, 38001 Santa Cruz de Tenerife (ES)
(72) Inventor: BRAVO GARCÍA, Pedro Luis, 38001 Santa Cruz de Tenerife (ES)
(74) Representative: Isern Patentes y Marcas S.L.
(86) International application number: PCT/ES2020/070319
(87) International publication number: WO 2021/234183

(56) References cited:
- WO-A1-2006/125986
- WO-A2-2010/091440
- US-A1- 2010 051 035
- US-A1- 2013 146 062
- US-A1- 2017 281 966
- US-A1- 2020 030 560
- US-B1- 6 745 773

## Description

### TECHNICAL SECTOR

The present invention can be included within the sector of instruments and devices for surgical use. In particular, the object of the invention relates to an endotracheal device used in mechanical ventilation of patients as an interface between patient and ventilation machine.

### BACKGROUND OF THE INVENTION

The mechanical ventilation of patients (3), either under anesthesia (in surgical or diagnostic procedures) or in critical situations (emergencies or intensive care), requires an interface between patient (3) and ventilation machine, which is currently embodied as masks of various designs and in endotracheal tubes (1), which have been used for more than 100 years and whose design has undergone only minor variations, but not changed essentially. This means that the risks and complications derived from the use thereof have remained constant during this long period of time.

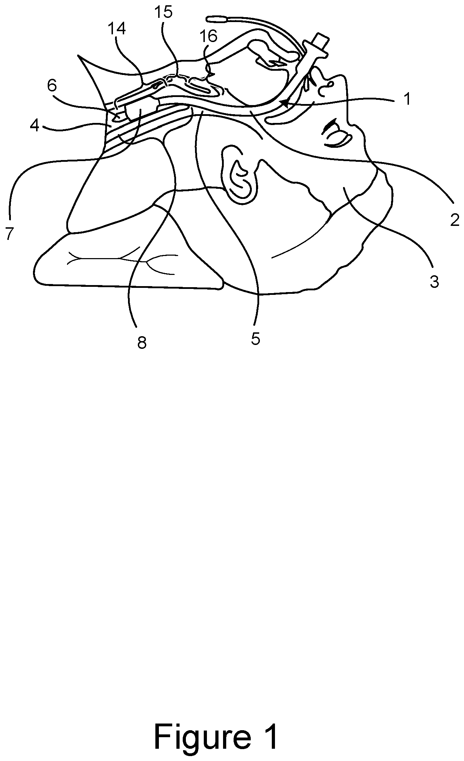

Figure 1 illustrates the use of one of said endotracheal tubes (1) of the state of the art. Said endotracheal tube (1) comprises a duct (2) which, during general anesthesia and once the patient (3) is asleep, is inserted through the mouth or the nose of the patient (3) until reaching the trachea (4) in the subglottic region. Said endotracheal tube (1) is connected to a ventilation machine, also called a respirator, or the like (not represented in said figure 1), the function of which is to keep the patient (3) breathing. Even if the duct (2) is correctly positioned, sometimes part of the stomach contents can pass to the lung of the patient (3) and cause severe respiratory problems. Similarly, with surgical interventions taking place in the pharynx and in the oral or nasal cavities, there is a risk that secretions or blood produced in the upper airway (above the glottis) will pass into the lower airway. To minimize this risk, the use of gauze tamponade as a barrier is indicated.

Airway lesions occur during anesthesia administered daily at any hospital. Vocal cord or laryngeal lesions are reflected in the post-operative period: sore throat (odynophagia), changes in the voice (dysphonia due to irritation of the vocal tract) or loss of voice (aphonia). Lesions that affect certain parts of the vocal cords, for example, the arytenoids, cause exhaustion of the voice throughout the day.

The emergence of videolaryngoscopy, together with the improvement in the reversal of muscle relaxants envisages an increase in the indications for intubations, increasing safety for patients (3). This is why it is necessary to have new devices that, while maintaining ventilation and anesthesia, provide greater safety and fewer complications and thus drastically improve the current situation.

The possibility of an epidemic with germs that lodge in the pulmonary tract indicates that the airway is increasingly secured, preferably avoiding the use of devices that do not provide good sealing in the future.

Likewise, this device enables patients to be ventilated more safely, reducing the possibility of contagion, both for the patient and for the healthcare professionals involved, in a case that requires ventilation in times of an epidemic.

Documents US6745773B1 (GOBEL), US2010/051035A1 (JENKINS), US2013/146062A1 (SCHUMACHER), WO2010/091440A2 (BHATT), US2020/030560A1 (JENKINS), US2017/281966A1 (BASIONY) and WO2006/125986A1 (LARYNGEAL MASK) disclose several models of tracheal cuffs.

### DESCRIPTION OF THE INVENTION

The present invention solves the aforementioned problems by means of an endotracheal device for mechanical ventilation of a patient, which ensures greater safety and a lower incidence of complications in mechanical ventilation of patients.

The endotracheal device of the invention comprises a ventilation tube and a barrier, wherein the ventilation tube includes: a distal part, with a distal end, intended to be inserted into an airway of a patient, and a proximal part, with a proximal end, intended to be connected to a ventilation machine.

Moreover, the barrier is made of solid viscoelastic material, covers the ventilation tube in the distal area, and is configured to occupy, when inserted, the subglottic region, as well as the glottic region and also the supraglottic region of the larynx of the patient, for which reason the barrier has a variable cross section along the length thereof. As will be explained later, the barrier has a triple effect, since it acts on the three above mentioned regions: subglottic, glottic and supraglottic.

In a more preferred example, the barrier comprises two frustoconical portions that come together in a cylindrical junction, reproducing the different diameters at the level of the trachea, vocal cords and supraglottic region, in a way that enables adaptation with a better treatment to the adjacent structures, as well as damping any movement made by the patient, such as change of position, seizure, etc. In accordance with the foregoing, the inclusion of the barrier provides triple protection: in the vocal cords, in the trachea and in the supraglottic region.

### BRIEF DESCRIPTION OF THE DRAWINGS

As a complement to the description, and for the purpose of helping to make the features of the invention more readily understandable, in accordance with a practical preferred exemplary embodiment thereof, said description is accompanied by a set of drawings which, by way of illustration and not limitation, represent the following.
Figure 1 shows a schematic view of an intubated patient by means of an endotracheal tube of the state of the art.
Figure 2 shows a life-size perspective view of an exemplary embodiment of the endotracheal device of the invention.
Figure 3 shows a schematic view of an intubated patient by means of the device of the invention.
Figure 4 shows a view of the triple effect barrier in an exemplary embodiment of the invention for the particular case of a pediatric patient.

### PREFERRED EMBODIMENT OF THE INVENTION

A detailed description of a preferred exemplary embodiment of an endotracheal device for mechanical ventilation of a patient (3), according to the present invention, is provided below with the help of figures 1-4 referred to above.

The endotracheal device of the invention constitutes a triple-barrier transglottic device, as explained later, and it can be abbreviated as ETT.

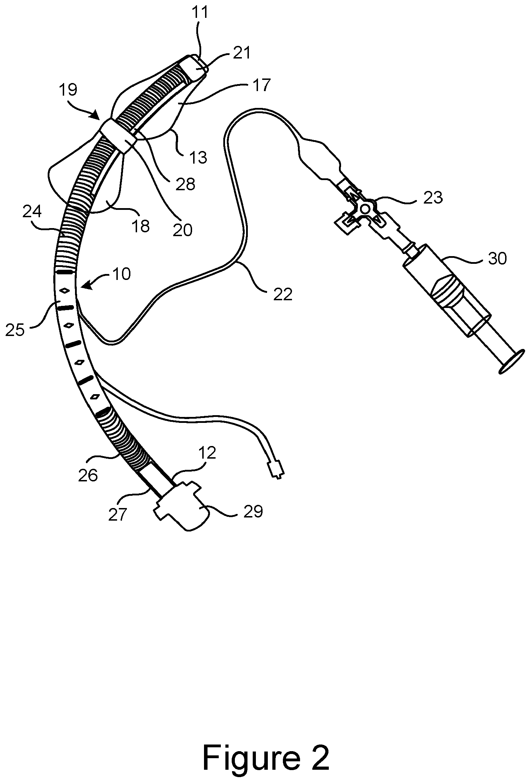

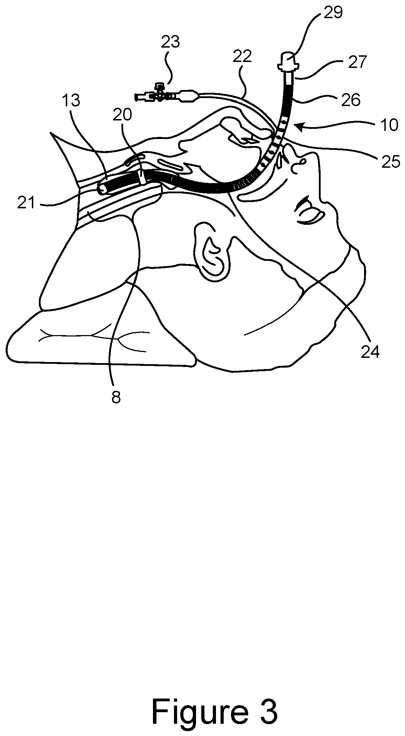

Figures 2 and 3 provide a life-size perspective view of a preferred embodiment of the endotracheal device. The endotracheal device comprises a ventilation tube (10) with a distal part, provided with a distal end (11), and a proximal part, provided with a proximal end (12), wherein the distal part is intended to be inserted into an airway of a patient (3), while the proximal part is intended to be connected to a ventilation machine (not shown).

Figures 1 and 3 show that the ventilation tube (10) is covered with a barrier (13) made of deformable solid material with viscoelastic behavior, arranged in the distal part of the ventilation tube (10), and intended to occupy, when inserted, the subglottic region (14), as well as the glottic region (15) and also the supraglottic region (16) of the larynx (5) of the patient (3). In the example shown, the barrier (13) occupies approximately the most distal third of the ventilation tube (10). The barrier (13) has dimensions, both in length and in transverse cross section, suitable for adapting to the morphology of the aforementioned subglottic region (14), glottic region (15) and supraglottic region (16). In particular, the barrier (13) has a variable transverse cross section along the length thereof. The barrier (13) preferably has symmetry of revolution, such that the transverse cross sections of the barrier (13) are defined at each point by the value of the corresponding diameter.

The different cross section/diameter values of the barrier (13) are derived from an anatomical study of the trachea in patients (3) and/or cadavers.

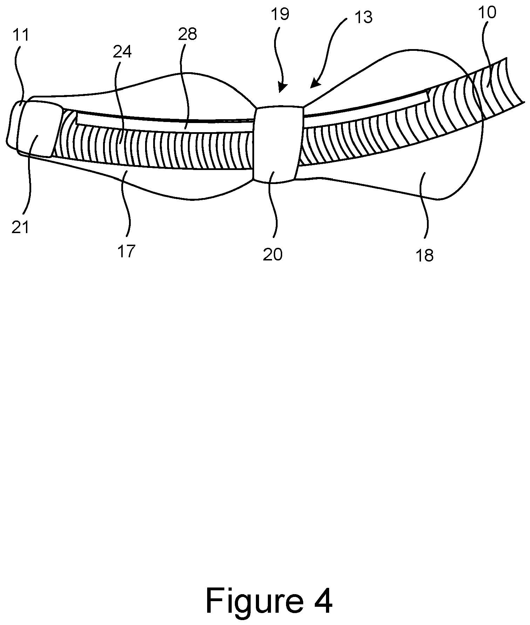

In a more distal location, the barrier (13) has a first portion (17), preferably with a straight or convex (not concave) longitudinal cross section, such that, for example, the first portion (17) has a conical shape, to adapt to the subglottic region (14). The configuration of the first portion (17) can vary for pediatric patients (3) with respect to adult patients (3). For adult patients, according to an exemplary embodiment, see figures 2 and 3, the first portion (17) has an increasing transverse cross section (or diameter) from the distal end (11), as well as preferably larger and smaller end diameters of 29 mm and 19 mm, respectively, to adequately adapt to the morphology of the subglottic region (14), which generally has an anteroposterior diameter of 14 to 18 mm and a transverse diameter of 18 to 22 mm (internal distances; the external distances are greater). The diameter differential between the first portion (17) of the barrier (13) and the subglottic region (14) of the larynx (5) of the patient (3) is established so that sufficient pressure is generated to seal and protect the airway at atmospheric pressure. On the other hand, for pediatric patients (3), in particular up to the age of nine, see figure 4, the first portion (17) can have an increasing transverse cross section (or diameter) from the distal end (11), wherein the larger and smaller end diameters have narrower dimensions than in the case of adults. In any case, the ventilation tube (10) and also the barrier (13), in particular the first portion (17) of the barrier (13), can be manufactured with different dimensions, without loss of generality or loss of performance, based on the morphology of different patients (3), both pediatric and adult.

Figures 2 and 3 also show that the barrier (13) comprises, in a more proximal location, a second portion (18), opposite from the first portion (17), with a transverse cross section (or diameter) increasing from the first portion (17), preferably with a straight or convex (not concave) longitudinal cross section, such that, for example, the second portion (18) has a conical shape, to adapt to the supraglottic region (16). According to an exemplary embodiment, the second portion (18) has smaller and larger end diameters of 19 mm and 34 mm, respectively, for reasons similar to those explained for the first portion (17) of the barrier (13).

Continuing with figures 2 and 3, they show a junction (19) of the first portion (17) with the second portion (18) of the barrier (13), intended to occupy the glottic region (15) at the level of the vocal cords and, according to the preferred example described, it has a well-known diameter of 19 mm, since the maximum opening of the vocal cords is generally 10 to 15 mm (at rest, 6-7 mm in males, 1-2 mm in females). More generally, the junction (19) has a preferred length comprised between 4 mm and 15 mm, as well as a preferred diameter comprised between 3 mm and 24 mm.

Preferably, as shown in figures 1 and 3, a placement mark (20) is provided, preferably opaque and more preferably black, located at the junction (19) of the first portion (17) with the second portion (18), intended to be located at the level of the vocal cords of the patient (3) and, therefore, the glottic region (15).

By way of example, in the preferred embodiment described, the barrier (13) has a total length of 10.5 cm, of which 5.5 cm correspond to the first portion (17) and 5 cm correspond to the second portion (18). More generally, the first portion (17) may have a preferred length of between 10 mm and 65 mm. Likewise, the second portion (18) can have a preferred length of between 10 mm and 75 mm.

Figures 2 and 3 show the location of each portion (17, 18) of the barrier (13) in the airway once the device has been inserted into the patient (3), wherein the subglottic region (14) is below the vocal cords, the glottic region (15) is at the level of the vocal cords and the supraglottic region (16) is above the vocal cords and up to the base of the epiglottis.

Figures 2 and 3 show that, at the distal end (11), preferably there is no Murphy eye (6), see figure 2, while there is a check mark (21), with a radio-opaque nature, which enables, once the device is inserted into the patient (3), the correct position of the distal part of the endotracheal device and, therefore, of the endotracheal device as a whole, to be checked by radiography.

The configuration of the device, both in shape and in size, is determined by the anatomical ranges presented by the larynx (5), i.e., epiglottis, cords, arytenoids, etc., the trachea (4) and the pharynx of the patients (3).

As the barrier (13) is composed of a deformable solid material with viscoelastic behavior, it is possible to insert the barrier (13) into the patient (3) in a collapsed state, i.e., compressed on itself, applying negative pressure on the barrier (13) and, once inserted, releasing said compression, or even applying positive pressure. In short, the device of the invention can include pressure means that can be connected to the barrier (13) to deform the barrier (13) due to the effect of fluid pressure and depression. This can be carried out, for example, by connecting to the barrier (13) a flexible conduit (22) in turn connected to a stopcock valve (23), such as, for example, a three-way stopcock valve (23), and aspirating by syringe (30) from the three-way stopcock valve (23). Once the device is placed inside the patient (3) and with the use of the aforementioned three-way stopcock valve (23), it will be possible to either let the ambient air pass freely towards the barrier (13), thereby achieving inflation at atmospheric pressure, or to apply positive pressure by injecting air with the syringe (30). The barrier (13) can be adapted to tracheostomy cannulas.

Figures 2 and 3 illustrate an arrangement of reinforced sections and unreinforced sections in the ventilation tube (10), as explained below. In the state of the art, reinforced, also called armored, endotracheal tubes (1) are known, which are characterized by having a spring-shaped inner reinforcement for exhaling, housed within the tube, to prevent the tension from any connection or movement from being transmitted along the entire device, as well as to prevent the tube from bending easily. However, if the patient (3) bites one of these reinforced tubes when they wake up from anesthesia or while they need mechanical ventilation, the reinforced tube will become deformed and will make ventilation difficult due to the increase in resistance subsequent to the decrease in size. Sometimes crushing is such that it prevents secretions from being sucked through it. These aforementioned drawbacks are avoided by using an unreinforced tube. In order to have the advantages associated with the reinforced tubes and the unreinforced tubes in a single tube, the device of the invention has alternating reinforced segments (24, 26) and unreinforced segments (25, 27) in the ventilation tube (10). In general, the ventilation tube (10) is mostly reinforced, with the advantages already mentioned, and unreinforced in the area where the patient (3) can bite the ventilation tube (10). In particular, the ventilation tube (10) has a first reinforced segment (24) located in the distal part, followed by a first unreinforced segment (25), located in an area that the patient (3) can bite, and then followed by a second reinforced segment (26), already in the proximal part. Preferably, a second unreinforced segment (27) that extends to the proximal end (12) follows it. The described combination of reinforced segments (24, 26) with unreinforced segments (25, 27) is a relevant novelty of the present invention.

Preferably, the ventilation tube (10) houses, in the distal part thereof, in at least part of the area covered by the barrier (13), a disinfecting radiation emitter (28) that emits, for example, ultraviolet (UV) radiation, for example, of type C, between 200-280 nm. Preferably, the disinfecting radiation emitter (28) comprises optical fiber that runs internally through the ventilation tube (10) and is sheathed in opaque material, being free of the opaque material in the area corresponding to the barrier (13), to emit radiation, for example UV radiation, in said area. The disinfecting radiation emitter (28), in particular the optical fiber, can reach, where appropriate, the radio-opaque check mark (21).

At the proximal end (12), the ventilation tube (10) can incorporate an indicator (29) that provides information about the diameter value of the ventilation tube (10), to facilitate a safe and quick connection to the ventilation machine. Preferably, the indicator (29) can be a cap. Even more preferably, the indicator (29), in particular the cap, has a color indicative of the diameter value, according to a pre-established color classification. Color classification allows for safer and faster availability of these devices, especially important in emergency situations where time is of the essence. This simple classification enables the required tube to be quickly located, which is not possible currently. According to an illustrative example, for diameters of 6.0, 6.5, 7.0, 7.5 and 8.0 mm, the colors pink, blue, red, green and yellow are used, respectively.

Preferably, the barrier (13), at least in an outermost layer, is made of partially permeable polymer (for example, nylon 6), to be impregnated with medically active substances, such as antiseptics, local anesthetics, etc.

Likewise, both the ventilation tube (10), either internally and/or externally, and the barrier (13) are preferably impregnated with nanoparticles (such as silver, Ag-Np, and zinc oxide, ZnO-Np), to protect against bacteria.

In this way, the stiffer parts of the device are never in contact with the most delicate structures of the respiratory tract of the patient (3): larynx (5) and trachea (4), which considerably reduces the possibility of damage to these valuable structures. Similarly, they make it easier for the patient (3) to adapt to the device during periods of waking up from anesthesia or weaning from mechanical ventilation. All this prevents (or at least minimizes) the appearance of typical airway lesions during anesthesia (sore throat or voice disorders, such as odynophagia, dysphonia and aphonia).

On the other hand, the length and extension of the barrier (13) confer greater protection against contamination of the airway of the patient (3) along the outer face of the device. The greater length means it is more difficult for any contaminated substance or secretion to access the interior due to the simple fact that the distance to be travelled by the contaminant is greater, much greater than that of the balloons (7) of the conventional endotracheal tubes (1) of the state of the art shown in figure 1, which, in addition, do not respect the anatomy of the structures. By considering the role of the glottic and supraglottic spaces as fundamental barriers in a novel way, two other points that hinder contamination of the airway can be established. The anatomical adaptation allows for better sealing.

The use of the device of the invention avoids the need to perform the pharyngeal tamponade referred to in the background, since the airway has already been originally isolated from the supraglottic region (16). Therefore, it is not necessary to perform gauze tamponades which, in addition to failing to achieve proper sealing, causes irritation of the pharynx.

Moreover, the device enables mechanical ventilation to be maintained for periods of time significantly longer than the current ones by drastically reducing the incidence of airway stenosis and avoiding or reducing indications of tracheostomy. In the case of prolonged intubation using the endotracheal tube (1) of the state of the art (see also figure 1), there is a high incidence in the appearance of tracheal stenosis, which increases the morbidity of patients (3) and, occasionally, mortality. This is due to the following reasons:
- The balloon (7) of the endotracheal tubes (1) on the walls of the trachea (4) is pushed on a very short segment of the trachea (4).
- Inflation pressure values of the balloon (7) higher than the capillary perfusion pressure at which the trachea (4) is being perfused.
- The site of the trachea (4) where the endotracheal tube rests and causes stenosis is known as the decubitus area (9), see figure 2. Not infrequently, this requires performing a tracheostomy, which is a surgical procedure consisting of establishing an artificial and invasive opening of the trachea (4) and channeling the trachea (4) from the outside. Said procedure is necessary from the 8^{th} -10^{th} day of mechanical ventilation, to avoid stenosis in the trachea (4). However, tracheostomy can also lead to complications: tracheal ulcers, false pathway, hemorrhages, infections, soft tissue distortion, obstruction of the airway, etc. This technique, however, does not prevent the incidence of post-tracheostomy stenosis from being high: 20-30 %.

The capillary blood pressure necessary for normal performance in the trachea (4) is 20 to 30 mm Hg, but this is highly variable and depends on the resistance and the condition of the patient (3). Therefore, to maintain the perfusion of the trachea (4), it is necessary to distribute the stresses generated by increasing the contact surface, with which the barrier (13) of the device of the invention effectively collaborates.

Given the length of the barrier (13), it is normally not necessary to apply positive pressures that can damage the trachea (4). What is more: if it is necessary to apply positive pressure, a very reduced pressure would be required which, moreover, would exert a very reduced real push on the airway, since the applied pressure has been distributed along the surface of the barrier (13), which is much greater than that of the balloons (7) of the conventional endotracheal tubes (1) (see also figure 1).

The comparison between the contact surfaces of an endotracheal tube (1) according to the state of the art (see figure 1) and of the barrier (13) of the device of the invention (figure 2) can be observed.

The main origin of tracheal stenosis is the resting of cannulas or tubes on the rear wall of the trachea, where the most vulnerable area is located since it is where the trachea (4) is nourished as it is the non-fibrotic part. This is damaged by being in contact with the endotracheal tube (1) in the decubitus area (8). If it is compared to figure 3, it is observed that no damage occurs in the decubitus area (8) due to the use of the barrier (13).

Sometimes the use of the endotracheal tubes (1) of the state of the art actively or passively causes bronchoaspiration, as well as intestinal reflux. This is because the airway is not completely protected or sealed. As the supraglottic region (16) is not protected and the balloons (7) of the endotracheal tubes (1) are short and, therefore, there is a small surface of interaction with the walls of the trachea (4), the possibilities that intestinal or oral germs pass through the site and cause contamination of the airway are high. Infections associated with mechanical ventilation currently represent an increase in the days of stay in intensive care units and significantly increase mortality in these patients (3). Furthermore, these nosocomial infections are usually caused by multi-resistant germs that are difficult to eradicate.

Normally, in patients (3) who require prolonged intubation, an invasive surgical procedure called a tracheostomy, which is not without complications and which drives up costs and diverts resources, will be indicated around day 8^{th}-10^{th}. In addition, many times this preventive tracheostomy is performed but soon after mechanical ventilation is removed from the patient (3), such that the procedure (viewed in hindsight) could have been avoided. Despite trying to obtain methods that predict who would need this preventive intervention, these methods fail and finally a tracheostomy is performed (as a lesser evil) before the tracheal stenosis lesion occurs. The device of the invention increases the days in which it is possible to maintain orotracheal ventilation, which would avoid many of the current tracheostomies.

There are two routes of contamination that lead to the dreaded nosocomial pneumonia associated with mechanical ventilation: the outer and inner faces of the endotracheal tubes (1). As explained above, the present invention provides three solutions:
- Greater length and contact surface of the barrier (13) with the airway in the three regions: subglottic (14), glottic (15) and supraglottic (16).
- Possibility of impregnating the barrier (13) with silver or zinc oxide nanoparticles, or with antiseptics or other substances, preventing the formation of bacterial biofilm and creating an active barrier against the infectious invasion of the airway through the outer face of the device.
- Possibility of emitting short-wave ultraviolet radiation (UVC 200-280 nm) in the distal part, which confers protection against contamination of the airway by the inner face of the device.

These possibilities will be desirable in epidemic situations with germs that have a high capacity to infect by air, since it protects not only the patient but also the personnel who are exposed to the patient.

Moreover, these solutions reduce the possibility of contamination of the airway. If the mechanical ventilation needs are short-term (less than 24 hours), the last two solutions could be ignored and thus reduce production costs.

Thanks to the configuration of the distal part, the device very significantly reduces the possibility that selective pulmonary ventilation occurs. Once the device is properly placed, the distance between the point that must rest on the vocal cords and the most distal point thereof is 5.5 cm according to the preferred example. In the endotracheal tubes (1) of the state of the art, this distance is 9.0 cm; therefore, it can touch and damage the tracheal carina or cause selective intubation, with the consequent complications: increased pressure in the respiratory tract, bronchospasm, increased anesthetic requirements, extrasystoles, etc.

The trachea (4) is highly variable in length (in fact, it does not always have the same number of rings); it ranges from 7 to 15 cm. Additionally, neck movements can cause a decrease in the length by contracting 3-4 rings. Thus, colliding against the tracheal carina or carrying out selective pulmonary intubation is avoided with the device of the invention. For reasons of increased safety, the Murphy eye (6), present in the endotracheal tubes (1), is preferably not considered.

The device of the invention makes it possible to reduce nosocomial pneumonia and infections associated with handling the airway.

The possibility of impregnating the barrier (13) with oral anesthetics enables the patient (3) to better adapt to the device during and at the end of anesthetic procedures. With endotracheal tubes (1) it is very common that the endotracheal tube (1) is rejected upon waking up from anesthesia (due to laryngeal reflexes). Impregnation with anesthetic leads to greater acceptance and tolerance by the patient (3).

The device of the invention provides better tolerance of the patient (3) to mechanical ventilation and to weaning from the same in long-term ventilation processes. Likewise, thanks to the check mark (21) located at the distal end, and absent in the endotracheal tubes (1), it is easier and clearer to locate it in the patient (3) through radiography.

## Claims

1. An endotracheal device for mechanical ventilation of a patient (3), the endotracheal device comprising a ventilation tube (10) comprising:
- a distal part, with a distal end (11), intended to be inserted into an airway of a patient (3),
- a proximal part, with a proximal end (12), intended to be connected to a ventilation machine; and
- a barrier (13) that covers the ventilation tube (10) in the distal area, and that it is configured to, when inserted, occupy, and adapt to, the morphology of the subglottic region (14), as well as the glottic region (15) and also the supraglottic region (16) of the larynx (5) of the patient (3), for which the barrier (13) has a variable cross section along the length thereof;
the endotracheal device further comprising pressure means connectable to the barrier (13) to deform the barrier (13),
the endotracheal device being **characterized in that** the barrier (13) is made of a solid viscoelastic material and comprises:
- in a more distal location, a first portion (17), with a transverse cross section increasing from the distal end (11), to adapt to the subglottic region (14); and
- in a more proximal location, a second portion (18), opposite from the first portion (17), with a transverse cross section increasing from the first portion (17), to adapt to the supraglottic region (16);
wherein the pressure means comprises a flexible conduit (22) connected to the barrier (13) and to an air stopcock valve (23), so as to apply a negative pressure for collapsing the barrier (13) before inserting the barrier into the patient (3) and, once inserted, releasing said compression.

2. The endotracheal device, according to claim 1, **characterized in that** the barrier (13) runs along the most distal third of the ventilation tube (10).

3. The endotracheal device, according to any of claims 1-2, **characterized in that** the barrier (13) has symmetry of revolution.

4. The endotracheal device, according to claim 1, **characterized in that** the first portion (17) of the barrier (13) has a straight or convex longitudinal cross section.

5. The endotracheal device, according to claim 4, **characterized in that** the first portion (17) has a conical shape.

6. The endotracheal device, according to claim 1, **characterized in that** the second portion (18) of the barrier (13) has a straight or convex longitudinal cross section.

7. The endotracheal device, according to claim 6, **characterized in that** the second portion (18) has a conical shape.

8. The endotracheal device, according to any of claims 1,6 and 7 **characterized in that** the first portion (17) and the second portion (18) come together in a junction (19) intended to occupy the glottic region (15) of the patient (3).

9. The endotracheal device, according to any of claims 1-8, **characterized in that** the barrier (13) incorporates a check mark (21), with a radio-opaque nature, at the distal end (11), to check the location of the endotracheal device in the patient (3).

10. The endotracheal device, according to any of claims 1-9, **characterized in that** the ventilation tube (10) comprises reinforced segments (24, 26) which comprise a spring-shaped inner reinforcement to prevent bending of the ventilation tube (10) and to prevent tension from any connection or movement from being transmitted along the ventilation tube (10), wherein the ventilation tube (10) further comprises an unreinforced segment (25) in correspondence with an area that can be bitten by the patient (3), so that the reinforced segments (24, 26) and the unreinforced segment (25) are alternating.

11. The endotracheal device, according to claim 10 **characterized in that** the ventilation tube (10) has a first reinforced segment (24) located in the distal part, followed by a first unreinforced segment (25), located in an area that the patient (3) can bite, and then followed by a second reinforced segment (26), already in the proximal part.

12. The endotracheal device, according to claim 11, **characterized in that** a second unreinforced segment (27), extending to the proximal end (12) follows the second reinforced segment (26).

13. The endotracheal device, according to any of claims 1-12, **characterized in that** the ventilation tube (10) comprises, in the distal part thereof, in at least part of the area covered by the barrier (13), a disinfecting radiation emitter (28).

14. The endotracheal device, according to claim 13, **characterized in that** the disinfecting radiation emitter (28) comprises optical fiber that runs internally through the ventilation tube (10) and is sheathed in opaque material, being free of the opaque material in the area corresponding to the barrier (13), to emit disinfecting radiation in said area.

## Patentansprüche

1. Endotrachealvorrichtung zur mechanischen Beatmung eines Patienten (3), wobei die Endotrachealvorrichtung einen Beatmungsschlauch (10) umfasst, Folgendes umfassend:
- einen distalen Teil mit einem distalen Ende (11), der dazu bestimmt ist, in einen Atemweg eines Patienten (3) eingeführt zu werden,
- einen proximalen Teil mit einem proximalen Ende (12), der dazu bestimmt ist, mit einem Beatmungsgerät verbunden zu werden; und
- eine Barriere (13), die den Beatmungsschlauch (10) in dem distalen Bereich abdeckt, und die ausgebildet ist, um, wenn sie eingeführt ist, die Morphologie der subglottischen Region (14) sowie der glottischen Region (15) und auch der supraglottischen Region (16) des Kehlkopfes (5) des Patienten (3) einzunehmen und sich daran anzupassen, wofür die Barriere (13) einen variablen Querschnitt entlang ihrer Länge aufweist;
wobei die Endotrachealvorrichtung ferner Druckmittel umfasst, die mit der Barriere (13) zum Verformen der Barriere (13) verbindbar sind,
wobei die Endotrachealvorrichtung **dadurch gekennzeichnet ist, dass** die Barriere (13) aus einem festen viskoelastischen Material besteht und Folgendes umfasst:
- an einem distaleren Ort, einen ersten Abschnitt (17) mit einem Querschnitt, der vom distalen Ende (11) aus zunimmt, um sich an die infraglottische Region (14) anzupassen; und
- an einem proximaleren Ort, einen zweiten Abschnitt (18), der dem ersten Abschnitt (17) gegenüberliegt, mit einem Querschnitt, der vom ersten Abschnitt (17) aus zunimmt, um sich an die supraglottische Region (16) anzupassen; wobei das Druckmittel eine flexible Leitung (22) umfasst, die mit der Barriere (13) und einem Luftabsperrventil (23) verbunden ist, um einen Unterdruck zum Kollabieren der Barriere (13) vor dem Einführen der Barriere in den Patienten (3) anzuwenden und nach dem Einführen die Kompression aufzuheben.

2. Endotrachealvorrichtung nach Anspruch 1, **dadurch gekennzeichnet, dass** die Barriere (13) entlang des distalsten Drittels des Beatmungsschlauchs (10) verläuft.

3. Endotrachealvorrichtung nach einem der Ansprüche 1-2, **dadurch gekennzeichnet, dass** die Barriere (13) Rotationssymmetrie aufweist.

4. Endotrachealvorrichtung nach Anspruch 1, **dadurch gekennzeichnet, dass** der erste Abschnitt (17) der Barriere (13) einen geraden oder konvexen Längsquerschnitt aufweist.

5. Endotrachealvorrichtung nach Anspruch 4, **dadurch gekennzeichnet, dass** der erste Abschnitt (17) eine konische Form aufweist.

6. Endotrachealvorrichtung nach Anspruch 1, **dadurch gekennzeichnet, dass** der zweite Abschnitt (18) der Barriere (13) einen geraden oder konvexen Längsquerschnitt aufweist.

7. Endotrachealvorrichtung nach Anspruch 6, **dadurch gekennzeichnet, dass** der zweite Abschnitt (18) eine konische Form aufweist.

8. Endotrachealvorrichtung nach einem der Ansprüche 1, 6 und 7, **dadurch gekennzeichnet, dass** der erste Abschnitt (17) und der zweite Abschnitt (18) in einer Verbindung (19) zusammenkommen, die dazu bestimmt ist, die glottische Region (15) des Patienten (3) einzunehmen.

9. Endotrachealvorrichtung nach einem der Ansprüche 1-8, **dadurch gekennzeichnet, dass** die Barriere (13) am distalen Ende (11) zur Überprüfung des Ortes der Endotrachealvorrichtung im Patienten (3) eine Prüfmarkierung (21) röntgendichter Beschaffenheit mit einbezieht.

10. Endotrachealvorrichtung nach einem der Ansprüche 1-9, **dadurch gekennzeichnet, dass** der Beatmungsschlauch (10) verstärkte Segmente (24, 26) umfasst, die eine federförmige Innenverstärkung umfassen, um zu verhindern, dass sich der Beatmungsschlauch (10) verbiegt, und um zu verhindern, dass Spannung aus einem Anschluss oder einer Bewegung entlang des Beatmungsschlauchs (10) übertragen wird, wobei der Beatmungsschlauch (10) ferner ein unverstärktes Segment (25) in Übereinstimmung mit einem Bereich umfasst, der von dem Patienten (3) gebissen werden kann, so dass die verstärkten Segmente (24, 26) und das unverstärkte Segment (25) sich abwechseln.

11. Endotrachealvorrichtung nach Anspruch 10, **dadurch gekennzeichnet, dass** der Beatmungsschlauch (10) ein erstes verstärktes Segment (24) aufweist, das sich im distalen Teil befindet, gefolgt von einem ersten unverstärkten Segment (25), das sich in einem Bereich befindet, auf den der Patient (3) beißen kann, und dann gefolgt von einem zweiten verstärkten Segment (26), das sich bereits im proximalen Teil befindet.

12. Endotrachealvorrichtung nach Anspruch 11, **dadurch gekennzeichnet, dass** ein zweites unverstärktes Segment (27), das sich bis zum proximalen Ende (12) erstreckt, auf das zweite verstärkte Segment (26) folgt.

13. Endotrachealvorrichtung nach einem der Ansprüche 1-12, **dadurch gekennzeichnet, dass** der Beatmungsschlauch (10) in seinem distalen Teil, mindestens in einem Teil des von der Barriere (13) abgedeckten Bereichs, einen desinfizierenden Strahlungsemitter (28) umfasst.

14. Endotrachealvorrichtung nach Anspruch 13, **dadurch gekennzeichnet, dass** der desinfizierende Strahlungsemitter (28) eine optische Faser umfasst, die im Inneren durch den Beatmungsschlauch (10) führt und mit undurchsichtigem Material ummantelt ist, wobei sie in dem der Barriere (13) entsprechenden Bereich frei von dem undurchsichtigen Material ist, um desinfizierende Strahlung in dem Bereich zu emittieren.

## Revendications

1. Dispositif endotrachéal pour la ventilation mécanique d'un patient (3), le dispositif endotrachéal comprenant un tube de ventilation (10) comprenant :
- une partie distale, avec une extrémité distale (11), destinée à être insérée dans les voies respiratoires d'un patient (3),
- une partie proximale, avec une extrémité proximale (12), destinée à être connectée à un appareil de ventilation ; et
- une barrière (13) qui recouvre le tube de ventilation (10) dans la zone distale, et qui est configurée pour, lorsque qu'elle est insérée, occuper, et s'adapter à, la morphologie de la région sous-glottique (14), ainsi qu'à la région glottique (15) et également à la région supraglottique (16) du larynx (5) du patient (3), pour lequel la barrière (13) a une section transversale variable sur sa longueur ;
le dispositif endotrachéal comprenant en outre un moyen de pression pouvant être connecté à la barrière (13) pour déformer la barrière (13),
le dispositif endotrachéal étant **caractérisé en ce que** la barrière (13) est constituée d'un matériau viscoélastique solide et comprend :
- dans un emplacement plus distal, une première partie (17), avec une section transversale augmentant à partir de l'extrémité distale (11), pour s'adapter à la région sous-glottique (14) ; et
- dans un emplacement plus proximal, une seconde partie (18), opposée à la première partie (17), avec une section transversale augmentant à partir de la première partie (17), pour s'adapter à la région supraglottique (16) ;
dans lequel le moyen de pression comprend une conduite souple (22) connectée à la barrière (13) et à un robinet d'arrêt d'air (23), de façon à appliquer une pression négative pour plier la barrière (13) avant d'insérer la barrière dans le patient (3) et, une fois insérée, relâcher ladite compression.

2. Dispositif endotrachéal, selon la revendication 1, **caractérisé en ce que** la barrière (13) court le long du tiers le plus distal du tube de ventilation (10).

3. Dispositif endotrachéal, selon l'une quelconque des revendications 1 à 2, **caractérisé en ce que** la barrière (13) a une symétrie de tour.

4. Dispositif endotrachéal, selon la revendication 1, **caractérisé en ce que** la première partie (17) de la barrière (13) a une section transversale longitudinale linéaire ou convexe.

5. Dispositif endotrachéal, selon la revendication 4, **caractérisé en ce que** la première partie (17) a une forme conique.

6. Dispositif endotrachéal, selon la revendication 1, **caractérisé en ce que** la seconde partie (18) de la barrière (13) a une section transversale longitudinale linéaire ou convexe.

7. Dispositif endotrachéal, selon la revendication 6, **caractérisé en ce que** la seconde partie (18) a une forme conique.

8. Dispositif endotrachéal, selon l'une quelconque des revendications 1, 6 et 7, **caractérisé en ce que** la première partie (17) et la seconde partie (18) se rejoignent en une jonction (19) destinée à occuper la région glottique (15) du patient (3).

9. Dispositif endotrachéal, selon l'une quelconque des revendications 1 à 8, **caractérisé en ce que** la barrière (13) incorpore une coche (21), de nature radio-opaque, à l'extrémité distale (11), pour vérifier l'emplacement du dispositif endotrachéal dans le patient (3).

10. Dispositif endotrachéal, selon l'une quelconque des revendications 1 à 9, **caractérisé en ce que** le tube de ventilation (10) comprend des segments renforcés (24, 26) qui comprennent un renfort interne en forme de ressort pour empêcher la flexion du tube de ventilation (10) et pour empêcher que la tension provenant d'une quelconque connexion ou d'un quelconque mouvement ne soit transmise le long du tube de ventilation (10), dans lequel le tube de ventilation (10) comprend en outre un segment non renforcé (25) en correspondance avec une zone qui peut être mordue par le patient (3), de sorte que les segments renforcés (24, 26) et le segment non renforcé (25) sont alternés.

11. Dispositif endotrachéal, selon la revendication 10, **caractérisé en ce que** le tube de ventilation (10) a un premier segment renforcé (24) placé dans la partie distale, suivi par un premier segment non renforcé (25), placé dans une zone que le patient (3) peut mordre, et ensuite suivi par un second segment renforcé (26), déjà dans la partie proximale.

12. Dispositif endotrachéal, selon la revendication 11, **caractérisé en ce qu'**un second segment non renforcé (27), s'étendant jusqu'à l'extrémité proximale (12), suit le second segment renforcé (26).

13. Dispositif endotrachéal, selon l'une quelconque des revendications 1 à 12, **caractérisé en ce que** le tube de ventilation (10) comprend, dans sa partie distale, dans au moins une partie de la zone recouverte par la barrière (13), un émetteur de rayonnement désinfectant (28).

14. Dispositif endotrachéal, selon la revendication 13, **caractérisé en ce que** l'émetteur de rayonnement désinfectant (28) comprend une fibre optique qui traverse de manière interne le tube de ventilation (10) et est gainée d'un matériau opaque, étant exempte du matériau opaque dans la zone correspondant à la barrière (13), pour émettre un rayonnement désinfectant dans ladite zone.
